# EUROPEAN PATENT APPLICATION

(11) **EP 4 681 704 A1**
(43) Date of publication of application: **21.01.2026**
(21) Application number: 24306216.3
(22) Date of filing: 18.07.2024
(51) Int. Cl.: A61K 9/20, A61K 31/4453, A61P 25/00, A61P 43/00, A61K 9/28

(54) **NEW FORMULATIONS OF PITOLISANT**

(71) Applicant: BIOPROJET Pharma, 75002 Paris (FR)
(72) Inventor: CAPET, Marc, 35520 Melesse (FR); ROBERT, Philippe, 38100 Grenoble (FR); FINANCE, Olivier, 35000 Rennes (FR); JULIEN, Jean-Stéphane, 37540 Saint Cyr sur Loire (FR); LECOMTE, Jeanne-Marie, 75003 Paris (FR); SCHWARTZ, Jean Charles, 75014 Paris (FR); MELSOPP, Elsie, 19462 Plymouth Meeting (US)
(74) Representative: Lavoix

(57) **Abstract**

The present disclosure relates to new formulations comprising an alkalinizing agent for improving absorption of active pharmaceutical ingredients, such as pitolisant.

## Description

The present invention concerns new formulations of active pharmaceutical ingredient such as pitolisant allowing improved absorption of said ingredient.

Pitolisant hydrochloride is a drug molecule that is useful for treating various diseases and disorders, particularly sleep disorders such as excessive daytime sleepiness (EDS) and cataplexy, that is marketed as WAKIX^{®}. WAKIX^{®} contains crystalline pitolisant hydrochloride.

Pitolisant is typically absorbed with a peak plasma concentration reached approximately three hours after administration and the plasmatic elimination half-life of pitolisant is 8 to 11 hours.

In order to achieve its awakening effect without affecting the nocturnal sleep of the patient, it is desirable that the effect of pitolisant is triggered as soon as possible after its administration. It is thus desirable to shorten the absorption time of pitolisant.

Doxepin hydrochloride was developed as an anti-depressant has also been approved in the treatment of insomnia. Its peak plasma concentration is reached 3.5 hours after administration. A quick onset of the effect is desired in order to promote sleep.

There is thus a need for shortening the time of absorption of active pharmaceutical ingredients, in particular active ingredients used for treating sleep disorders or diseases.

It has now been discovered that active pharmaceutical ingredients that are typically administered in the form of a chloride salt may be more rapidly absorbed if they are administered in combination with an alkalinizing agent.

In particular, it has been shown that the plasmatic absorption peak of pitolisant hydrochloride or doxepin hydrochloride occurs more rapidly when co-administered with an alkalinizing agent.

Accordingly, the present disclosure generally relates to a new use allowing *inter alliae* accelerated crossing of an active pharmaceutical ingredient through the intestinal barrier.

In particular, it relates to a combination of a salt of an active pharmaceutical ingredient with an alkalinizing agent.

### SUMMARY

In a first object, the present invention relates to an accelerated absorption formulation comprising an active pharmaceutical ingredient, in the form of a salt, together with an alkalinizing agent.

In another object, it also relates to the use of an alkalinizing agent to accelerate the absorption of an active pharmaceutical ingredient administered in the form of a salt.

In embodiments, said salt may be a hydrochlride salt.

In embodiments, active pharmaceutical ingredients administered in the form of a salt, such as hydrochloride particularly relate to pitolisant or doxepine.

The present invention particularly concerns a combination of a pitolisant salt such as pitolisant hydrochloride with an alkalinizing agent.

Still further, it also relates to a pharmaceutical composition comprising the combination of salt of pitolisant such as pitolisant hydrochloride and an alkalinizing agent as defined above, and optionally one or more pharmaceutically acceptable excipients.

### DETAILED DESCRIPTION

The invention relates to combinations of an active pharmaceutical ingredient in the form of a pharmaceutically acceptable salt, preferably hydrochloride salt, together with an alkalinizing agent.

More specifically, it relates to combinations of pitolisant in the form of a pharmaceutically acceptable salt, preferably hydrochloride salt, with an alkalinizing agent.

It also relates to combinations of doxepine in the form of a pharmaceutically acceptable salt, preferably hydrochloride salt, with an alkalinizing agent.

### Active Pharmaceutical ingredients

As used herein, this term particularly refers to pharmaceutically active compounds that are formulated in the form of an acid addition salt such as with hydrochloric acid.

In an embodiment, the disclosure relates to pitolisant.

In an embodiment, the disclosure relates to doxepin.

### Salts

Salts typically refer to pharmaceutically acceptable salts, in particular acid addition salts. Such salts are generally used to achieve a crystalline and/or solid form of a compound that may be otherwise amorphous and/or liquid.

In embodiments, salts are hydrochloride salts.

Such hydrochloride acid addition salts are typically considered to be pharmaceutically acceptable.

They can be obtained by contacting the neutral form of said active pharmaceutical ingredient with a sufficient amount of hydrochloric acid, either neat or in a suitable solvent (*e.g.*, an inert solvent). Procedures are to prepare such salts are known to those of skill in the art.

### Pitolisant

The term "pitolisant hydrochloride" refers to the addition salt of pitolisant of the following formula :

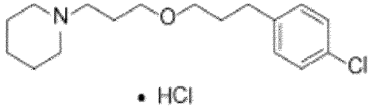

It is herein to be understood that this term encompasses its various amorphous or crystalline forms such as its polymorphic forms including its form I (disclosed *inter alliae* in U.S. Patent No. 8,207,197 that is incorporated herein by reference in its entirety) and its form II (disclosed in U.S. Patent application No. 18/621,972 and PCT/FR2024/050398 that are also incorporated herein by reference in their entirety).

### Doxepin

The term "doxepin hydrochloride" refers to the addition salt of doxepin of the following formula :

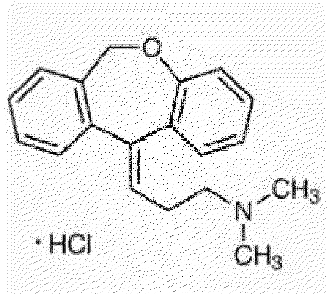

### The alkalinizing agent

As used herein, an alkalinizing agent is an agent able to increase the pH. It is typically a salt, such as chosen from magnesium, calcium and sodium salts together with the suitable counter-ion.

According to an embodiment, counter-ions include carbonate, citrate, bicarbonate, acetate, lactate, oxide salts.

In embodiments, said alkalinizing agent include one or more of the following: tetraborate, hydroxide, oxide, carbonate, citrate, bicarbonate, acetate, lactate, arginine, lysine, choline, meglumine, thromethamine, potassium, magnesium, calcium and sodium salts
Preferably, said allkalinizing agent is chosen from magnesium carbonate, sodium carbonate, potassium bicarbonate, magnesium oxide, preferably magnesium carbonate.

### The combinations

The combinations of the invention typically exhibit a more rapid absorbtion of the active ingredient within the body than the active ingredient.

In particular, it has been shown that the plasmatic absorption peak of the active ingredient occurs more rapidly when co-administered with an alkalinizing agent.

Accordingly, the combinations of the invention allow an accelerated absorbtion (ie) a decrease of the so-called Tmax.

Tmax generally defines the minimum time to reach the peak plasma concentration of an active ingredient after administration (Cmax).

In embodiments, the Cmax of the combination may be more than 5%, preferably more than 10%, still more preferably more than 20% of the Cmax obtained with the corresponding active ingredient.

It is also believed that the combinations of the invention may allow higher absorption of the active ingredient.

Higher absorption is typically illustrated by a higher AUC (ie) a higher integral of the concentration-time curve.

In embodiments, the AUC of the combination may be more than 5%, preferably more than 10%, still more preferably more than 20% of the AUC obtained with the corresponding active ingredient.

In an embodiment, the weight ratio (alkalinizing agent/alkalinizing agent+pitolisant salt) is comprised between 5 and 99%, preferably between 15 and 95% more preferably between 15 and 70%.

According to an embodiment, said combination has a pH comprised between 7.5 and 11.

### Dosage Forms and Pharmaceutical Compositions

According to a further object, the present invention also relates to a pharmaceutical composition comprising a combination disclosed herein, and optionally one or more pharmaceutically acceptable excipients.

According to a further object, the present invention also relates to a dosage form comprising said pharmaceutical composition disclosed herein

Disclosed herein are dosage forms and pharmaceutical compositions comprising pitolisant salt, such as hydrochloride and an alkalinizing agent and optionally one or more pharmaceutically acceptable excipients.

Typically, said pharmaceutical composition or dosage form is in the form of a tablet. Typically, said tablet comprises (in weight) :
3 to 20% of pitolisant salt such as hydrochloride ;
2 to 50% alkalinizing agent;

With respect to the total weight of the coated tablet.

In embodiments, said tablet comprises a core coated by a film, wherein said core comprises pitolisant salt, such as hydrochloride, and said alkalinizing agent.

Typically, said core may further comprise one or more pharmaceutically acceptable excipients chosen from microcrystalline cellulose, magnesium stearate, anhydrous colloidal silica, lactose and croscarmellose sodium.

In an alternative, said pharmaceutical composition is in the form of a coated tablet, said coated tablet comprising:
A core comprising
   3 to 15% of pitolisant hydrochloride ;
   20 to 50% of alkalinizing agent;
   35 to 50% of pharmaceutically acceptable excipients chosen from microcrystalline cellulose, magnesium stearate, anhydrous colloidal silica and lactose; and
A coating film, said coating film representing 2 to 5% in weight,
   With respect to the total weight of the coated tablet.

Typically, said coating film may comprise one or more ingredients including polyvinyl alcohol, titanium oxide, macrogol and talc.

Suitable ready-to-use compositions for preparing such coating film (herein called "coating systems") are commercially available, such as the Opadry^{®} ambll coating system produced by Colorcon.

According to another alternative, the pharmaceutical composition may be in the form of a gastro-resistant tablet.

Accordingly, said gastro-resistant tablet comprises:
A core comprising :
   10 to 20% of pitolisant hydrochloride ;
   2 to 20% of alkalinizing agent;
   40 to 60% of pharmaceutically acceptable excipients chosen from microcrystalline cellulose, magnesium stearate, anhydrous colloidal silica, lactose and croscarmellose sodium;
A coating film comprising :
   10 to 15% of one or more gastro-resistant coating agents, with respect to the total weight of the coated tablet;
   2 to 5% of pharmaceutically acceptable excipients chosen from coating systems, plasticizers ;
   With respect to the total weight of the coated tablet.

As used herein « gastro-resistant coating agent" refers to a composition suitable for preparing a gastro-resistant film.

Suitable ready-to-use compositions for preparing such gastro-resistant coating film are commercially available, such as the Acryl EZE^{®} composition produced by Colorcon.

The dosage form or pharmaceutical composition can comprise a therapeutically effective amount of pitolisant salt. For example, the dosage form or pharmaceutical composition may comprise between about 1 mg and about 200 mg, *e*.*g*., between about 1 mg and about 100 mg, between about 1 mg and about 50 mg, between about 10 mg and about 25 mg, or between about 1 mg and about 10 mg, of pitolisant hydrochloride, *e*.*g*., about 3 mg, about 4 mg, about 5 mg, about 6 mg, about 8 mg, about 10 mg, about 12 mg, about 15 mg, about 20 mg, about 25 mg, about 30 mg, about 40 mg, or about 50 mg, of pitolisant hydrochloride. It will be understood that the amount of pitolisant in the form of the free base in the dosage form or pharmaceutical composition will be slightly lower than the equivalent amount as mentioned above. For example, a dosage form or pharmaceutical composition disclosed herein comprising 5 mg of pitolisant hydrochloride will comprise about 4.45 mg of pitolisant (freebase). In another example, a dosage form or pharmaceutical composition disclosed herein comprising 20 mg of pitolisant hydrochloride comprises about 17.8 mg of pitolisant (freebase). In some embodiments, a dosage form or pharmaceutical composition disclosed herein comprises about 5 mg of pitolisant monohydrochloride, or about 4.45 mg pitolisant (freebase). In some embodiments, a dosage form or pharmaceutical composition disclosed herein comprises about 20 mg of pitolisant monohydrochloride, or about 17.8 mg pitolisant (freebase).

The dosage forms of the present disclosure can be tablets, caplets, capsules, suspensions, granules, powders, or the like.

For example, the dosage form of said pharmaceutical composition is a tablet, more particularly a film-coated tablet.

According to an embodiment, said core comprises pitolisant hydrochloride and said alkalinizing agent.

The dosage forms or pharmaceutical compositions of the present disclosure can further comprise in addition to the hydrochloride salt of the active pharmaceutical ingredient and the alkalinizing agent, one or more pharmaceutically acceptable excipients, such as diluents, dispersing agents, granulating agents, surface active agents, emulsifiers, disintegrating agents (sometimes referred to herein as disintegrants), binding agents (sometimes referred to herein as binders), preservatives, buffering agents, lubricating agents (sometimes referred to herein as lubricants), glidants, adjuvants, fillers, wetting agents, suspending agents, solvents, dispersion media, ion exchangers, salts, electrolytes, waxes, and/or oils, and the like.

The pharmaceutical composition or dosage form may comprise a pharmaceutically acceptable excipient disclosed herein, or a combination of pharmaceutically acceptable excipients disclosed herein.

For example, a dosage form or pharmaceutical composition of the present disclosure may comprise one or more, or all of, the following pharmaceutically acceptable excipients: colloidal silicon dioxide, crospovidone, magnesium stearate, microcrystalline cellulose, polyethylene glycol, polyvinyl alcohol, talc, and titanium dioxide.

Each pharmaceutically acceptable excipient can be present in the dosage form or pharmaceutical composition in any suitable amount. For example, a pharmaceutically acceptable excipient can be present in the dosage form or pharmaceutical composition in an amount of between about 0.01% and about 95% by weight of the dosage form or pharmaceutical composition, e.g., between about 0.1% and about 25%, between about 1% and about 10%, between about 15% and about 95%, between about 0.01% and about 2%, by weight of the dosage form or pharmaceutical composition.

Dosage forms and pharmaceutical compositions of the present disclosure may be administered orally, parenterally, by inhalation, topically, rectally, nasally, buccally, vaginally, or by implantation.

Preparation of the dosage forms or pharmaceutical composition of the present disclosure can include conventional methods, such as blending, filling, compressing (*e*.*g*., direct compression, compression of dry, wet or sintered granules), coating (*e.g*., coating in a spray process), extrusion, granulation (*e.g.*, wet or dry granulation), pelleting (*e.g.*, direct pelleting), binding, powder layering (*e*.*g*., onto active ingredient-free beads, or neutral cores or particles of pharmaceutically active agent), and rounding off.

### Methods of Treatment

In another object, the present invention also relates to a combination, dosage form or a pharmaceutical composition disclosed herein, for use for treating a disease or disorder such as those disclosed herein,

According to a further object, the present invention also concerns a method a method for the treatment of a disease or disorder, comprising administering said combination, or a dosage form or pharmaceutical composition disclosed herein, to a subject in need thereof.

### Methods of Treatment where the active pharmaceutical ingredient is a pitolisant:

The disease or disorder may be a sleep disorder (*e*.*g*., excessive daytime sleepiness (EDS), cataplexy, narcolepsy, sleep apnea (e.g., obstructive sleep apnea), sleep induced apnea, diurnal somnolence), severe fatigue, idiopathic hypersomnia central nervous system disorder (*e.g.*, epilepsy, Alzheimer's disease, Parkinson's disease, dementia (*e.g.*, dementia with Lewy bodies and/or vascular dementia), attention disorders, wakefulness disorders, memorization disorders, cognitive deficits (*e*.*g*., in aged persons), psychiatric pathologies, depressive and asthenic states, vertigo, and motion sickness), obesity, psychosomatic disorders, respiratory disorders, allergic conditions, inflammatory conditions, cardiac conditions, gastrointestinal conditions, conditions of the urogenital system, conditions of the cutaneous system, stress, migraine, headache, pain, psychotropic disorders, asthma, bronchitis, rhinitis, tracheitis, gastric ulcers, duodenal ulcers, ulcerative colitis, Crohn's disease, irritable bowel syndrome (IBS), cystitis, metritis, urinary incontinence, fecal incontinence, urticaria, itching, arthritis, conjunctivitis, premenstrual syndrome, prostatic inflammations, genital disorders, rheumatic conditions, ocular conditions, sialorrhea, convulsion, depression, disorders of the hypothalamo-hypophyseal system, disorders of the cerebral circulation, and disorders of the immune system.

The disease or disorder may be typically a sleep disorder. For example, the disease or disorder may be excessive daytime sleepiness (EDS), cataplexy, narcolepsy, sleep apnea (e.g., obstructive sleep apnea), sleep induced apnea, diurnal somnolence, severe fatigue or idiopathic hypersomnia.

In some embodiments, the disease or disorder is excessive daytime sleepiness (EDS). In some embodiments, the disease or disorder is cataplexy. In a method disclosed herein, the subject to be treated may have narcolepsy, and/or may be an adult with narcolepsy. For example, the method of treating a disease or disorder may comprise administering to a subject in need thereof a combination or pharmaceutical composition of the invention.

In another object, the present invention also relates to a combination of pitolisant salt with an alkalinizing agent, or a pharmaceutical composition disclosed herein, for use in the treatment of a disease or disorder (*e*.*g*., a disease or disorder disclosed herein), optionally, wherein the disease or disorder is excessive daytime sleepiness (EDS), cataplexy, narcolepsy, sleep apnea (*e*.*g*., obstructive sleep apnea), sleep induced apnea, diurnal somnolence, severe fatigue or idiopathic hypersomnia.

The disease or disorder may be in a subject with narcolepsy (*e*.*g*., an adult subject with narcolepsy).

In another object, the present invention also relates to the use of a combination of pitolisant salt with an alkalinizing agent, or a pharmaceutical composition disclosed herein, for the manufacture of a medicament for the treatment of a disease or disorder (*e*.*g*., a disease or disorder disclosed herein), optionally, wherein the disease or disorder is excessive daytime sleepiness (EDS), cataplexy, narcolepsy, sleep apnea (*e*.*g*., obstructive sleep apnea), sleep induced apnea, diurnal somnolence, severe fatigue or idiopathic hypersomnia.

The disease or disorder can be in a subject with narcolepsy (*e*.*g*., an adult subject with narcolepsy).

The present disclosure further relates to a method for the prevention of undesirable side effects associated with using antipsychotic or antidepressant agents (*e*.*g*., aripiprazole, clozapine, olanzapine, risperidone, quetiapine, sertindole, mirtazapine, amitryptiline, and paroxetine), comprising administering the combination, or a dosage form or pharmaceutical composition of the present disclosure, to a subject in need thereof. Non-limiting examples of undesirable side effects associated with using antipsychotic or antidepressant agents includes weight gain, somnolence, and cognitive impairment.

The present disclosure further relates to a method for (i) inducing an extended state of wakefulness; (ii) improving cognitive processes; (iii) reducing food intake; and/or (iv) normalizing vestibular reflexes, comprising administering the combination, or a dosage form or pharmaceutical composition disclosed herein, to a subject in need thereof.

The combination, or a dosage form or pharmaceutical composition disclosed herein, may be administered once daily, twice daily, or more often. More than one dosage form can be administered at once to achieve a desired dose. The combination, or a dosage form or pharmaceutical composition disclosed herein, may be taken with a frequency and in such an amount so that the total amount of pitolisant (in terms of freebase) administered is within the range of from about 10 mg to about 50 mg per day, *e*.*g*., about 15 mg to about 40 mg per day. The combination, or a dosage form or pharmaceutical compositions disclosed herein, may be taken with a frequency and in such an amount so that the total amount of pitolisant (in terms of freebase) administered is within the range of from about 17.8 mg to about 35.6 mg per day. For example, a subject may be administered orally two dosage forms once daily, where each dosage form comprises 4.45 mg pitolisant (in terms of freebase), to achieve a daily dose of 8.9 mg pitolisant (in terms of freebase). A subject may be administered orally one dosage form once daily, where the dosage form comprises 17.8 mg pitolisant (in terms of freebase), to achieve a daily dose of 17.8 mg pitolisant (in terms of freebase). A subject may be administered orally two dosage forms once daily, where each dosage form comprises 17.8 mg pitolisant (in terms of freebase), to achieve a daily dose of 35.6 mg pitolisant (free base).

### Methods of Treatment where the active pharmaceutical ingredient is doxepin:

The disease or disorder include depressive disorders, anxiety disorders, chronic hives, insomnia and itchiness.

### Use for improving drug absorption

According to an object, the invention provides for improving absorption of an active ingredient, said use comprising administering said active ingredient in the form of a salt together withan alkalinizing agent, such as defined above.

As used herein, "improving absorption" particularly refers to accelerated absorption of the active ingredient within the patient's body. It may also refer to higher absorption within the patient's body.

According to a further object, the invention also concerns an accelerated absorption formulation comprising an active pharmaceutical ingredient in the form of a salt combined with an alkalinizing agent.

### Definitions

The articles "a" and "an" are used herein to refer to one or more than one (*i.e.*, to at least one) of the grammatical object of the article. By way of example, "an element" means one element or more than one element.

The term "about" when referring to a measurable value such as an amount, a temporal duration, and the like, is meant to encompass variations of ±20% or less, or in some instances ±15% or less, or in some instances ±10% or less, or in some instances ±5% or less, or in some instances ±1% or less, or in some instances ±0.1% or less, from the specified value, as such variations are appropriate.

The phrase "and/or" as used herein should be understood to mean "either or both" of the elements so conjoined, *i.e.*, elements that are conjunctively present in some cases and disjunctively present in other cases. Multiple elements listed with "and/or" should be construed in the same fashion, *i.e.*, "one or more" of the elements so conjoined. Other elements may optionally be present other than the elements specifically identified by the "and/or" clause, whether related or unrelated to those elements specifically identified. Thus, as a non-limiting example, a reference to "A and/or B," when used in conjunction with openended language such as "comprising" can refer, in one embodiment, to A only (optionally including elements other than B); in another embodiment, to B only (optionally including elements other than A); in yet another embodiment, to both A and B (optionally including other elements); etc.

The terms "administer," "administering," or "administration," as used herein refer to implanting, absorbing, ingesting, injecting, inhaling, or otherwise introducing a compound, dosage form, or pharmaceutical composition.

The terms "comprise," "comprises," and "comprising" are used herein in a non-exclusive sense, except where the context requires otherwise. Likewise, the term "include" and its grammatical variants are intended to be non-limiting, such that recitation of items in a list is not to the exclusion of other like items that can be substituted or added to the listed items. The term "effective amount" or a "therapeutically effective amount" as used herein refers to an amount of a compound, or a pharmaceutical composition, described herein, which is sufficient to achieve a desired result under the conditions of administration. For example, an effective amount of a compound, dosage form, or pharmaceutical composition disclosed herein for treating excessive sleep disorder (EDS), *e.g.*, in a subject with narcolepsy, is an amount that can reduce the effects of the EDS, and/or reduce or eliminate the severity of a symptom associated with the EDS. A skilled clinician can determine appropriate dosing based on a variety of considerations including the severity of the disease, the subject's age, weight, general health and other considerations.

The term "pharmaceutically acceptable excipient" as used herein refers to a non-toxic material that may be formulated with a compound disclosed herein (*e*.*g*., pitolisant) to provide a pharmaceutical composition. Preferably, the pharmaceutically acceptable excipient is inert and does not interfere with the pharmacological activity of a compound which it is formulated with. Pharmaceutically acceptable excipients useful in the manufacture of the pharmaceutical compositions disclosed herein are any of those well known in the art, and include without limitation, diluents, dispersing agents, granulating agents, surface active agents, emulsifiers, disintegrating agents (sometimes referred to herein as disintegrants), binding agents (sometimes referred to herein as binders), preservatives, buffering agents (sometimes referred to herein as buffers), lubricating agents (sometimes referred to herein as lubricants), glidants, adjuvants, fillers, wetting agents, suspending agents, solvents, dispersion media, ion exchangers, salts, electrolytes, waxes, and/or oils, and the like.

For example, a pharmaceutically acceptable excipient may be alumina, a phosphate (*e*.*g*., calcium phosphate, dicalcium phosphate, tricalcium phosphate, disodium hydrogen phosphate, potassium hydrogen phosphate), a sulfate (*e*.*g*., calcium sulfate), a cellulose, kaolin, bentonite, lactose, mannitol, sorbitol, sucrose, inositol, compressible sugar, trehalose, xylitol, acacia, gelatin, glucose, maltodextrin, starch (*e*.*g*., corn starch, potato starch), sodium starch glycolate, starch derivatives, an amino acid, polyvinylpyrrolidone (PVP, povidone) (*e*.*g*., crosslinked PVP, crospovidone), polyvinyl alcohol, tragacanth, polyethylene glycol, mineral clay powders, croscarmellose, poloxamer, fatty acids or salts thereof (*e*.*g*., lauric acid, sodium lauryl sulfate, stearic acid, calcium stearate, magnesium stearate, aluminum stearate, oleic acid), hydrogenated vegetable oils, talc, titanium dioxide, glyceryl behenate, silicon dioxide (*e*.*g*., colloidal silicon dioxide), a silicate salt (e.g., magnesium trisilicate), lecithin, serum protein (*e*.*g*., human serum albumin), sorbic acid, potassium sorbate, a metal cation salt (*e*.*g*., a sodium salt, such as sodium chloride, a potassium salt, such as potassium chloride, a magnesium salt, such as or magnesium chloride, a zinc salt, such as zinc chloride), water, dimethylacetamide, protamine sulfate, wool fat, ethylenediaminetetraacetic acid (EDTA), a cyclodextrin (*e*.*g*., CAPTISOL^{®}), a polysorbate (*e.g.*, TWEEN^{®}, *e.g.*, TWEEN^{®} 20 or TWEEN^{®} 80), and combinations thereof. The term "pharmaceutically acceptable salt" as used herein refers to salts of a compound prepared with relatively nontoxic acids or bases, depending on the particular substituents found on the respective compound. When compounds contain relatively basic functionalities, acid addition salts can be obtained by contacting the neutral form of such compounds with a sufficient amount of the desired acid, either neat or in a suitable solvent (*e.g.*, an inert solvent). Examples of pharmaceutically acceptable acid addition salts include those derived from inorganic acids like hydrochloric acid, hydrobromic acid, nitric acid, carbonic acid, monohydrogencarbonic acid, phosphoric acid, monohydrogenphosphoric acid, dihydrogenphosphoric acid, sulfuric acid, monohydrogensulfuric acid, hydriodic acid, or phosphorous acids and the like, as well as the salts derived from organic acids like acetic acid, propionic acid, isobutyric acid, maleic acid, malonic acid, benzoic acid, succinic acid, suberic acid, fumaric acid, lactic acid, mandelic acid, pamoic acid, phthalic acid, benzenesulfonic acid, p-toluenesulfonic acid, citric acid, tartaric acid, methanesulfonic acid, oxalic acid, and the like. Also included are salts of amino acids such as arginate and the like, and salts of organic acids like glucuronic or galacturonic acids and the like. Other pharmaceutically acceptable salts known to those of skill in the art are suitable for pharmaceutical compositions relating to the present disclosure.

The term "solvate" as used herein refers to forms of a compound that are associated with a solvent, usually by a solvolysis reaction. This physical association may include hydrogen bonding. Conventional solvents include water, methanol, ethanol, acetic acid, dimethyl sulfoxide (DMSO), tetrahydrofuran (THF), diethyl ether, and the like. Compounds of the present disclosure may be prepared, *e*.*g*., in crystalline form, and may be solvated. Suitable solvates include pharmaceutically acceptable solvates and further include both stoichiometric solvates and non-stoichiometric solvates. In certain instances, the solvate will be capable of isolation, for example, when one or more solvent molecules are incorporated in the crystal lattice of a crystalline solid. "Solvate" encompasses both solution-phase and isolable solvates. Representative solvates include hydrates, ethanolates, and methanolates.

The term "hydrate" as used herein refers to a compound which is associated with water. Typically, the number of the water molecules contained in a hydrate of a compound is in a definite ratio to the number of the compound molecules in the hydrate. Therefore, a hydrate of a compound may be represented, for example, by the general formula R•xH₂O, wherein R is the compound and wherein x is a number greater than 0. A given compound may form more than one type of hydrate, including, *e*.*g*., monohydrates (x is 1), lower hydrates (x is a number greater than 0 and smaller than 1, *e*.*g*., hemihydrates (R•0.5H₂O)), and polyhydrates (x is a number greater than 1, *e*.*g*., dihydrates (R•2H₂O) and hexahydrates (R•6H₂O)).

The term "subject" as used herein refers to any animal, such as any mammal, including but not limited to, humans, non-human primates, rodents, dogs, and the like. Non-human primates include chimpanzees, cynomolgus monkeys, spider monkeys, and macaques (*e*.*g*., Rhesus). Rodents include mice, rats, woodchucks, ferrets, rabbits, and hamsters. Domestic and game animals include cows, horses, pigs, deer, bison, buffalo, feline species (*e.g.*, domestic cat), canine species (*e.g.*, dog, fox, wolf), avian species, and fish. In some embodiments, the subject is a mammal (*e*.*g*., a human, a rat, or a mouse). The subject can be male or female. The subject may be of any age, including an elderly human subject (*e*.*g*., 65 years or older), a human subject that is not elderly (*e*.*g*., less than 65 years old), or a human pediatric subject (*e*.*g*., less than 18 years old). In preferred aspects, the subject is a human.

As used herein, the terms "treat," "treatment," "treating," or grammatically related terms, refer to a method of reducing the effects of a disease or disorder. As is readily appreciated in the art, full eradication of the disease, disorder, or symptoms thereof is preferred but not a requirement for treatment. Desirable effects of treatment include, but are not limited to, preventing occurrence or recurrence of the disease or disorder, alleviation of symptoms, diminishment of any direct or indirect pathological consequences of the disease or disorder, or other improvement of any sign, symptom, or consequence of the disease or disorder, such as prolonged survival, less morbidity, and/or a lessening of side effects.

Throughout this disclosure, various embodiments can be presented in a range format (*e*.*g*., from X to Y). It should be understood that the description in range format is merely for convenience and brevity and should not be construed as an inflexible limitation on the scope of the present disclosure. Accordingly, the description of a range should be considered to have specifically disclosed all the possible subranges as well as individual numerical values within that range. For example, description of a range, such as from 1 to 6, should be considered to have specifically disclosed subranges such as from 1 to 5, from 1 to 4, from 1 to 3, from 2 to 6, from 2 to 4, from 3 to 6, etc., as well as individual numbers within that range, e.g., 1, 2, 2.8, 3, 3.6, 4, 5, 5.4, and 6. As another example, a range such as 95-99% includes 95%, 96%, 97%, 98%, or 99% and all subranges such as 96-99%, 96-98%, 96-97%, 97-99%, 97-98%, etc. This applies regardless of the breadth of the range

All publications (*e.g*., scientific journal articles, patent publications, and the like) cited in this disclosure are incorporated by reference in their entirety. To the extent the material incorporated by reference contradicts or is inconsistent with this specification, the specification will supersede any such material. The citation of any references herein is not an admission that such references are prior art to the present disclosure. Various terms relating to aspects of the description are used throughout the specification and claims. Such terms are to be given their ordinary meaning in the art unless otherwise indicated. Other specifically defined terms are to be construed in a manner consistent with the definitions provided herein.

Compounds (*e*.*g*., pharmaceutically active agents) disclosed herein may also comprise one or more isotopic substitutions. For example, hydrogen (H) may be in any isotopic form, including ¹H, ²H (D or deuterium), ³H (T or tritium); carbon (C) may be in any isotopic form, including ¹²C, ¹³C, and ¹⁴C; oxygen (O) may be in any isotopic form, including ¹⁶O and ¹⁸O; nitrogen (N) may be in any isotopic form, including ¹⁴N and ¹⁵N; and chlorine (Cl) may be in any isotopic form, including ³⁵Cl and ³⁷Cl.

Various embodiments of the combinations, pharmaceutical compositions, and methods herein are described in further detail below, and additional definitions may be provided throughout the specification.

### BRIEF DESCRIPTION OF DRAWINGS

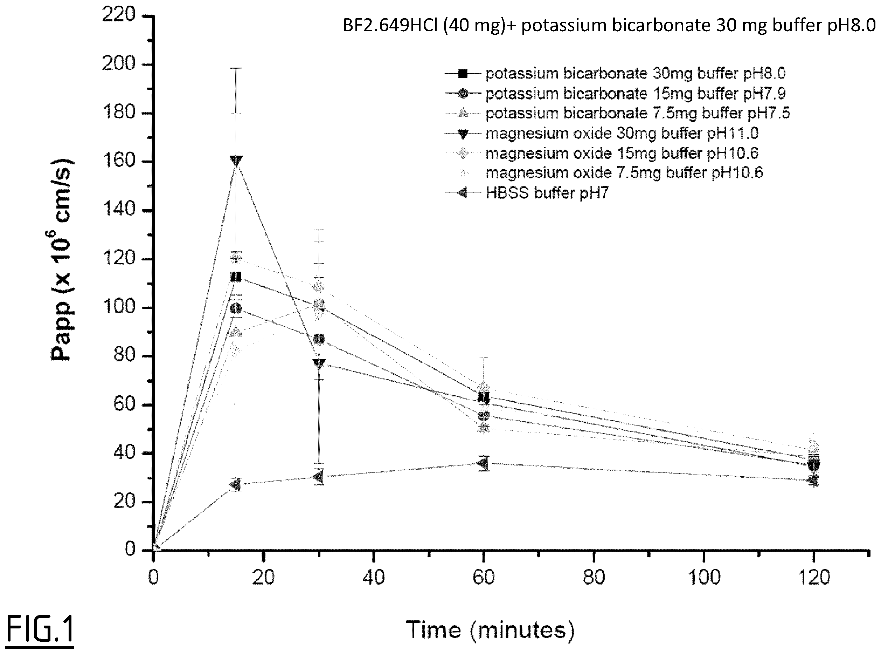

**FIGURE 1****,** **FIGURE 2** **and** **FIGURE 3** are graphical representations of the kinetics of various combinations of pitolisant hydrochloride (40 mg).

In Figure 1, the apparent permeability (Papp) (10⁶ cm/s) over time (120 min) is illustrated for some representative combinations having various pH, said pH being further adjusted with a buffer.
pitolisant hydrochloride 40 mg/ potassium bicarbonate 30 mg - pH 8.0
pitolisant hydrochloride 40 mg/ potassium bicarbonate 15 mg - pH 7.9
pitolisant hydrochloride 40 mg/ potassium bicarbonate 7.5 mg - pH 7.5
pitolisant hydrochloride 40 mg/ magnesium oxide 30 mg - pH 11.0
pitolisant hydrochloride 40 mg/ magnesium oxide 15 mg - pH 10.6
pitolisant hydrochloride 40 mg/ magnesium oxide 7.5 mg - pH 10.6

The results show that the combinations of pitolisant hydrochloride with an alkalinizing agent involve an early peak, together an increased permeability compared to control (pitolisant hydrochloride with buffer).

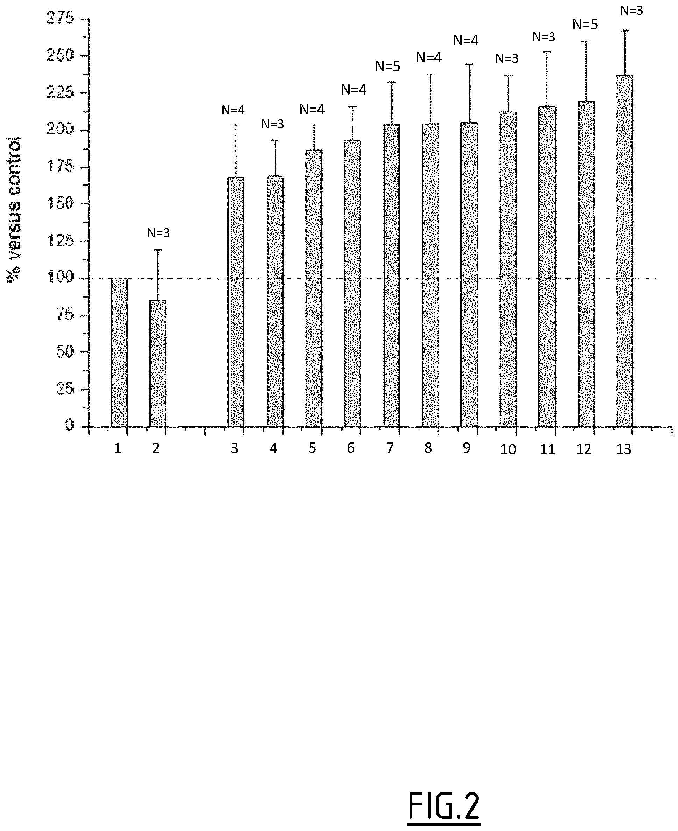

**Figure 2** illustrates the bioavailability (area under the curve, AUC, 0-120 min) (expressed as % over control) for some representative combinations of pitolisant hydrochloride having various pH:
1: Control (pitolisant hydrochloride 40 mg/HBSS buffer pH 7)
2: pitolisant hydrochloride 40 mg pH 8
3: pitolisant hydrochloride 40 mg/potassium bicarbonate 7.5 mg pH 7.5
4: pitolisant hydrochloride 40 mg/sodium carbonate 7.5 mg pH 8.6
5: pitolisant hydrochloride 40 mg/potassium bicarbonate 15 mg pH 7.9
6: pitolisant hydrochloride 40 mg/sodium carbonate 30 mg pH 9.5
7: pitolisant hydrochloride 40 mg/magnesium oxide 30 mg pH 11
8: pitolisant hydrochloride 40 mg/magnesium carbonate 30 mg HBSS pH 9.8
9: pitolisant hydrochloride 40 mg/magnesium carbonate 15 mg HBSS pH 9.9
10: pitolisant hydrochloride 40 mg/potassium bicarbonate 30 mg pH 8
11: pitolisant hydrochloride 40 mg/magnesium oxide 7.5 mg pH 9.8
12: pitolisant hydrochloride 40 mg/magnesium carbonate 7.5 mg HBSS pH 9.7
13: pitolisant hydrochloride 40 mg/magnesium oxide 15 mg pH 10.6

The results show that the combinations of pitolisant hydrochloride with an alkalinizing agent lead to a higher bioavailability compared to control (pitolisant hydrochloride with buffer).

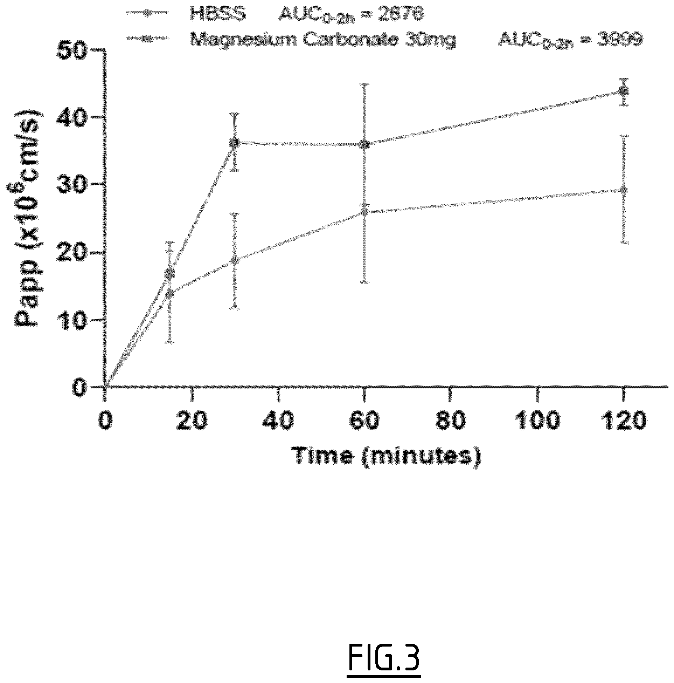

In Figure 3, the apparent permeability (Papp) (10⁶ cm/s) of doxepine hydrochloride over time (120 min) is illustrated for some representative combinations:
doxepine hydrochloride 10 mg/HBSS
doxepine hydrochloride 10 mg/magnesium carbonate 30 mg pH 7.5
Nous avons les resultats expérimentaux du passage dans les Caco2 de la doxépine.

The results show that the combinations of doxepine hydrochloride with an alkalinizing agent lead to a higher bioavailability (+50% over 2 hours) compared to doxepine hydrochloride with buffer (Hnak's balanced salt solution, HBSS).

### EXAMPLES

### Illustrative formulations

4 formulations of pitolisant hydrochloride (40 mg) with different alkalinizing agents are administered orally to patients:
Treatment A: 30 mg magnesium carbonate
Treatment B: 7.5 mg magnesium carbonate
Treatment C: 30 mg potassium bicarbonate
Treatment D: 30 mg sodium carbonate.

Each tablet is coated with a gastro-resistant film.

| **Raw material** | **Unit formula (mg/unit)** | **Unit formula (mg/unit)** | **Unit formula (mg/unit)** | **Unit formula (mg/unit)** |
|---|---|---|---|---|
| **Formulation** | **Treatment A** | **Treatment B** | **Treatment C** | **Treatment D** |
| Pitolisant HCl | 40.00^{a} | 40.00^{a} | 40.00^{a} | 40.00^{a} |
| Magnesium carbonate | 30.00 | 7.50 | / | / |
| Potassium bicarbonate | / | / | 30.00 | / |
| Sodium carbonate | / | / | / | 30.00 |
| Lactose | 107.17 | 122.17 | 107.17 | 107.17 |
| Microcrystalline cellulose | 53.58 | 61.08 | 53.58 | 53.58 |
| Croscarmellose sodium | 11.25 | 11.25 | 11.25 | 11.25 |
| Anhydrous colloidal silica | 2.00 | 2.00 | 2.00 | 2.00 |
| Magnesium stearate | 6.00 | 6.00 | 6.00 | 6.00 |
| **TOTAL** | **250.0** | **250.0** | **250.0** | **250.0** |

| **Coating** | | | | |
|---|---|---|---|---|
| Opadry amb II¹ (clear, 88A190022) | 7.5 | 7.5 | 7.5 | 7.5 |
| Acryl Eze² 93A19346 clear | 38.625 | 38.625 | 38.625 | 38.625 |
| PEG 8000³ | 3.09 | 3.09 | 3.09 | 3.09 |
| **TOTAL** | **299.215** | **299.215** | **299.215** | **299.215** |

| | | | | |
|---|---|---|---|---|
| ^{a} Salt / Base ratio = 1.1234 - corresponding to 35.6 mg of pitolisant base. ¹ 15% suspension, weight gain = 3% ² 20% suspension, weight gain = 15% ³ PEG at 8% of Acryl Eze | | | | |

Pitolisant doses are expressed as pitolisant-HCl dose in this protocol. 40 mg pitolisant-HCl dose corresponding to 35.6 mg pitolisant base.

## Claims

1. A combination of a salt of pitolisant with an alkalinizing agent.

2. The combination according to claim 1 where said pitolisant salt is pitolisant hydrochloride.

3. Combination according to claim 1 or 2 wherein said alkalinizing agent comprises one or more of the following tetraborate, hydroxide, oxide, carbonate, citrate, bicarbonate, acetate, lactate, arginine, lysine, choline, meglumine, thromethamine, potassium, magnesium, calcium and sodium salts.

4. Combination according to anyone of the preceding claims wherein said alkalinizing agent is magnesium carbonate and/or sodium carbonate, preferably magnesium carbonate.

5. The combination according to anyone of the preceding claims wherein the weight ratio (alkalinizing agent/alkalinizing agent+pitolisant salt) is comprised between 5 and 99%, preferably between 15 and 95% more preferably between 15 and 70%.

6. The combination according to anyone of the preceding claims for use for treating or preventing a disease or disorder chosen from the group consisting of excessive daytime sleepiness (EDS), cataplexy, narcolepsy, sleep apnea (e.g., obstructive sleep apnea), sleep induced apnea, diurnal somnolence, severe fatigue and idiopathic hypersomnia.

7. The combination for use according to anyone of the preceding claims for accelerated and/or higher absorption of pitolisant.

8. A pharmaceutical composition comprising the combination of a pitolisant salt and an alkalinizing agent as defined in anyone of the preceding claims, and optionally one or more pharmaceutically acceptable excipients.

9. The pharmaceutical composition according to claim 8 which is in the form of a tablet and wherein said tablet comprises (in weight) :
3 to 20% of pitolisant hydrochloride ;
2 to 50% alkalinizing agent;
With respect to the total weight of the coated tablet.

10. The pharmaceutical composition according to claim 8 or 9, wherein said tablet comprises a core coated by a film,
Said core comprising pitolisant hydrochloride and said alkalinizing agent.

11. The pharmaceutical composition according to claim 10 wherein the core further comprises one or more pharmaceutically acceptable excipients chosen from microcrystalline cellulose, magnesium stearate, anhydrous colloidal silica, lactose and croscarmellose sodium.

12. The pharmaceutical composition according to anyone of claims 8 to 11, which is in the form of a coated tablet, comprising:
A core comprising :
3 to 15% of pitolisant hydrochloride ;
20 to 50% of alkalinizing agent;
35 to 50% of pharmaceutically acceptable excipients chosen from microcrystalline cellulose, magnesium stearate, anhydrous colloidal silica and lactose;
A coating film, said coating film representing 2 to 5% in weight,
With respect to the total weight of the coated tablet.

13. The pharmaceutical composition according to anyone of claims 8 to 11, which is in the form of a gastro-resistant tablet, comprising:
A core comprising :
10 to 20% of pitolisant hydrochloride ;
2 to 20% of alkalinizing agent;
40 to 60% of pharmaceutically acceptable excipients chosen from microcrystalline cellulose, magnesium stearate, anhydrous colloidal silica, lactose and croscarmellose sodium;
A coating film comprising
10 to 15% of one or more gastro-resistant coating agents, with respect to the total weight of the coated tablet;
2 to 5% of pharmaceutically acceptable excipients chosen from coating systems, plasticizers;
With respect to the total weight of the coated tablet.

14. An accelerated absorption formulation comprising an active pharmaceutical ingredient in the form of a salt combined with an alkalinizing agent.

15. Use of an alkalinizing agent to accelerate the absorption of an active pharmaceutical ingredient comprising administering said active pharmaceutical ingredient in the form of a salt, together with said alkalinizing agent.

16. The accelerated absorption formulation according to claim 14 or use according to claim 15 wherein the active pharmaceutical ingredient is doxepin.

17. The accelerated absorption formulation according to claim 14 or use according to claim 15 wherein the active pharmaceutical ingredient is pitolisant.

18. The accelerated absorption formulation or use according to anyone of claims 14 to 17 wherein the alkalinizing agent is defined as in anyone of claims 2 to 4.
